(19) Europäisches Patentamt
European Patent Office
Office européen des brevets

(11) Veröffentlichungsnummer **0 056 932**
**B1**

(12) # EUROPÄISCHE PATENTSCHRIFT

(45) Veröffentlichungstag der Patentschrift: **19.09.84**

(51) Int. Cl.³: **C 07 D 301/14** // C07D303/04

(21) Anmeldenummer: **82100001.5**

(22) Anmeldetag: **02.01.82**

(54) **Verfahren zur Herstellung und Isolierung von n-Alkyl-oxiranen.**

(30) Priorität: **15.01.81 DE 3101037**
**15.01.81 DE 3101049**

(43) Veröffentlichungstag der Anmeldung: **04.08.82 Patentblatt 82/31**

(45) Bekanntmachung des Hinweises auf die Patenterteilung: **19.09.84 Patentblatt 84/38**

(84) Benannte Vertragsstaaten: **AT BE CH DE FR GB IT LI NL**

(56) Entgegenhaltungen:
**DE - A - 2 602 776**
**DE - A - 2 619 091**
**DE - A - 2 835 940**
**DE - B - 1 230 005**

(73) Patentinhaber: **BAYER AG, Konzernverwaltung RP Patentabteilung, D-5090 Leverkusen 1 Bayerwerk (DE)**

(72) Erfinder: **Rauleder, Gebhard, Dr., Mozartstrasse 20, D-5657 Haan (DE)**
Erfinder: **Waldmann, Helmut, Dr., Henry-T.-von Böttinger Strasse 15, D-5090 Leverkusen (DE)**

**EP 0 056 932 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft ein Verfahren zur Herstellung von Oxiranen durch Umsetzung von α-Olefinen mit Percarbonsäuren und Aufarbeitung der dabei erhältlichen Reaktionsgemische.

Oxirane finden Verwendung auf dem Gebiet der Lacke, zur Herstellung von Polyethern, Polyurethanen, Epoxidharzen, Detergentien, Glykolen und einer Vielzahl von organischen Zwischenprodukten (siehe beispielsweise US-PS 2 412 136 und DE-AS 1 139 477).

Es ist bekannt, daß man Oxirane nach der Chlorhydrinmethode durch Umsetzung von Olefinen mit Chlor im alkalischen Medium und nachfolgende Behandlung mit Basen herstellen kann. Ein grundsätzlicher Nachteil dieses Verfahrens besteht darin, daß salzhaltige umweltbelastende Abwässer und unerwünschte chlorierte Nebenprodukte gebildet werden (siehe Ullmann's Enzyklopädie der technischen Chemie, Band 10, Seite 565 (3. Auflage)).

Ein weiteres Syntheseprinzip beruht auf der Umsetzung von Olefinen mit organischen Hydroperoxiden in Gegenwart von Katalysatoren (siehe DE-AS 1 468 012). Diese Verfahren haben den entscheidenden Nachteil, daß das organische Hydroperoxid (ROOH, wobei R z. B. einen niedermolekularen Rest, wie t-Butyl oder Cumyl, bedeuten kann), gemäß nachstehender Gleichung während der Reaktion in den entsprechenden Alkohol (ROH) umgewandelt wird:

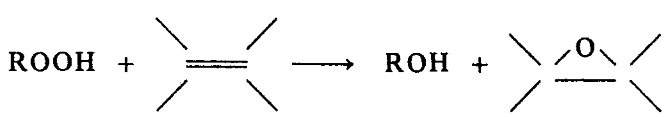

Es fällt somit der dem eingesetzten Hydroperoxid entsprechende Alkohol als Koppelprodukt an, was eine Abhängigkeit der Wirtschaftlichkeit dieses Verfahrens von der Verwertbarkeit dieses Alkohols mit sich bringt. Falls er nicht verwertet werden kann, muß er über mehrere Verfahrensstufen in das entsprechende Hydroperoxid zurückverwandelt werden, was besonderen technischen Aufwand erfordert.

Eine Möglichkeit, unter Umgehung dieser Nachteile Olefine in Epoxide umzuwandeln, besteht in der Anwendung der »Prileschajew-Reaktion« (vgl. N. Prileschajew, Ber. dtsch. chem. Ges. 42, 4811, (1909)). Bei dieser Reaktion handelt es sich um einen elektrophilen Angriff einer Percarbonsäure auf ein Olefin (vgl. K. D. Bingham, G. D. Meakins, G. H. Whitham, Chem. Commun. 1966, S. 445 und 446). Aus diesem Grunde nimmt die Reaktivität des Olefins mit fallender Nucleophilie der Doppelbindung ab.

α-Olefine zählen daher zu den schwer epoxidbaren Verbindungen, z. B. beträgt die relative Reaktivität von

$$Alk{-}CH{=}CH_2 \quad = \quad 25$$

und von    Alk = Alkyl

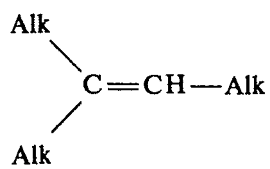

= 6000

(vgl. D. Swern »Organic Peroxides«, Wiley Interscience, Vol. II, S. 452).

Infolge der geringen Reaktionsfähigkeit der Doppelbindung in α-Olefinen sind oft lange Reaktionszeiten erforderlich, was zur Bildung von unerwünschten Nebenprodukten, wie Dihydroxy- und Hydroxyacyloxy-Derivaten der Ausgangsprodukte, Anlaß gibt (vgl. S. N. Lewis in R. L. Augustin »Oxidation«, Vol. 1, S. 233, Z. 6—11, Marcel Dekker, New York (1969)).

So werden bei der Umsetzung von $C_{12}$- bis $C_{18}$-Olefinen mit Peressigsäure bei einer Reaktionstemperatur von 25°C und Reaktionszeiten von 28 Stunden die Alkyloxirane in Ausbeuten von nur 40 bis 50% erhalten (vgl. D. Swern et. al., J. Am. Chem. Soc. 68, 1504—7 (1946)).

Zur Verbesserung dieser Ergebnisse wurden spezielle Methoden angewandt. So wurde in der DE-AS 1 230 005 ein Verfahren zur Herstellung von Alkyloxiranen publiziert, bei dem α-Olefine mit Peressigsäure im organischen Lösungsmittel umgesetzt werden, derart, daß man die Reaktionskomponente bis zu einem Peressigsäureumsatz von etwa 55 bis 80% der eingesetzten Menge bei einer Temperatur unterhalb 50°C behandelt, worauf man in einem oder mehreren Temperatursprüngen auf Temperaturen oberhalb etwa 50°C die Umsetzung bis zu einem maximalen Peressigsäureumsatz vervollständigt, und daß man am Ende der Reaktion das entstandene 1,2-Alkanepoxid vom Lösungsmittel und aus vorhandener Essigsäure durch Abdestillieren befreit.

Dabei wurden, wie im Beispiel 1, Spalte 4, für 1-Dodecen beschrieben, bei einem Persäure-Umsatz von 94%, 86 Mol-% 1,2-Dodecanepoxid und 6 Mol-% 1,2-Dodecanglykolmonoacetat erhalten. Wurde

die Reaktion isotherm bei 50°C durchgeführt, wie im Beispiel 2 beschrieben, wurden nach einer Reaktionszeit von 30 Stunden und 94%igem Persäure-Umsatz nur 74 Mol-% 1,2-Dodecanepoxid und 15 Mol-% 1,2-Dodecanglykolmonoacetat erhalten.

Neben der unbefriedigenden Ausbeute und der Bildung von erheblichen Mengen an Nebenprodukten ist ein weiterer Nachteil dieses Verfahrens, daß es nur dann möglich ist, einigermaßen akzeptable Ergebnisse zu erhalten, wenn man die Reaktion nach einem komplizierten Temperaturprogramm durchführt. Auch muß das gewünschte Epoxid vor der Destillation noch mit Wasser nachgewiesen werden (siehe Beispiel 1 der DE-AS 1 230 005), um die restliche Essigsäure zu entfernen, was technisch aufwendig und teuer ist, zu erheblichen Essigsäureverlusten führt und umweltbelastende Abwässer ergibt.

In der DE-OS 1 568 016 wird ein Epoxidierungsverfahren für langkettige $\alpha$-Olefine beschrieben, bei dem in einem zweiphasigen System mit in situ gebildeter Peressigsäure epoxidiert wird, wobei ein besonderes Kennzeichen des Verfahrens die Anordnung des Rührers und die Rührgeschwindigkeit darstellt. Der Rührvorgang darf nur so durchgeführt werden, daß es sich um ein »sanftes Rühren« handelt, und zwar dergestalt, daß lediglich die organische Phase dabei bewegt wird. Als bevorzugte Reaktionszeit werden 18 bis 20 Stunden angegeben, was die technische Anwendung dieser Methode teuer und unwirtschaftlich macht.

Die Verwendung von Monoperphthalsäure zur Epoxidation von Decen und Hexen wird in IZV. Akad. SSSR, 1966, Nr. 6, S. 1088—1089 beschrieben. Dabei werden die Olefine in etherischer Lösung bei 3 bis 20°C mit Monoperphthalsäure umgesetzt. Obwohl die Reaktion in Gegenwart von Puffersalzen, wie Natriumcarbonat, durchgeführt wird, resultieren die Alkyloxirane, z. B. bei der Umsetzung mit Decen-1, in Ausbeuten von nur 66%.

Auch m-Chlorperbenzoesäure wurde zur Epoxidation von $\alpha$-Olefinen eingesetzt (siehe US-PS 4 113 875). Im Beispiel 1 dieser Patentschrift ist die Methode, mit der sowohl 1-Alkene als auch 2-Alkene umgesetzt wurden, beschrieben. Dabei wurde bei einer Reaktionstemperatur von 15 bis 25°C und einer Reaktionszeit von 10 Stunden das Oxiran in einer Ausbeute von nur 76% erhalten. Neben der unbefriedigenden Ausbeute, ist auch die komplizierte Aufarbeitung des Reaktionsgemisches für eine technische Anwendung ungeeignet. Die überschüssige Persäure muß thermisch zerstört werden, was nicht nur unwirtschaftlich ist, sondern für eine technische Anwendung auch ein erhebliches Sicherheitsrisiko mit sich bringt. Das Reaktionsgemisch wird mit Salzlösungen gewaschen, was zu umweltbelastenden Abwässern führt.

Es wurde nun ein Verfahren zur Herstellung und Isolierung von Alkyloxiranen durch Umsetzung von Alkenen mit Percarbonsäuren gefunden, wobei anschließend das Reaktionsgemisch, gegebenenfalls nach einer Extraktion mit Wasser, durch Destillation in seine Komponenten aufgetrennt wird, das dadurch gekennzeichnet ist, daß man ein Alken der Formel

$$CH_3{-}(CH_2)_n{-}CH=CH_2$$

worin

n für eine ganze Zahl von 4 bis 17 steht,

mit einer Lösung einer 3 oder 4 C-Atome enthaltenden Percarbonsäure in einem organischen Lösungsmittel bei einer Temperatur im Bereich 40 bis 90°C und einem Molverhältnis von Alken zu Percarbonsäure von 0,9 : 1 bis 5 : 1 umsetzt, wobei die Percarbonsäurelösung unter 5 Gew.-% Wasser, unter 0,5 Gew.-% Wasserstoffperoxid und unter 50 ppm Mineralsäure enthält.

Als organisches Lösungsmittel können für die erfindungsgemäße Umsetzung von Alkenen mit einer 3 oder 4 C-Atome enthaltenden Percarbonsäure beispielsweise die verschiedensten unsubstituierten und substituierten Kohlenwasserstoffe verwendet werden, die unter den Reaktionsbedingungen flüssig sind und unerwünschte Nebenreaktionen nicht oder nur in ganz untergeordnetem Maße eingehen. Solche Kohlenwasserstoffe sind z. B. aliphatische und cycloaliphatische Kohlenwasserstoffe wie Hexan, Heptan, Octan, 2-Ethyl-hexan, Decan, Dodecan, Cyclohexan, Methylcyclopentan und Petrolether, aromtische Kohlenwasserstoffe wie Benzol, Nitrobenzol, Toluol, Ethylbenzol, Cumol, Diisopropylbenzol, Xylol und Chlorbenzol, sauerstoffhaltige Kohlenwasserstroffe wie Ether und Ester, z. B.

Diethylether, Diisopropylether, Dibutylether, Tetrahydrofuran, Dioxan, Essigsäureethylester, Essigsäuremethylester, Essigsäurepropylester, Essigsäurebutylester, Propionsäuremethylester, Propionsäureethylester, Propionsäurepropylester, Buttersäuremethylester, Buttersäureethylester, Buttersäurepropylester, Buttersäurebutylester, Benzoesäuremethylester und Benzoesäureethylester, chlorierte Kohlenwasserstoffe wie Methylenchlorid, Chloroform, Tetrachlorkohlenstoff, 1-Chlorethan, 1,2-Dichlorethan, 1,1-Dichlorethan, 1,1,2,2-Tetrachlorethan, 1-Chlorpropan, 2-Chlorpropan, 1,2-Dichlorpropan, 1, 3-Dichlorpropan, 2,3-Dichlorpropan, 1,2,3-Trichlorpropan, 1,1,2,3-Tetrachlorpropan, Butylchlorid, 1,2-Dichlorbutan, 1,4-Dichlorbutan, 2,3-Dichlorbutan, 1,3-Dichlorbutan, 1,2,3,4-Tetrachlorbutan, tert.-Butylchlorid, Amylchlorid, 1,2-Dichlorpentan, 1,5-Dichlorpentan, 1,2,3,4-Tetrachlorpentan, Cyclopentylchlorid, 1,

2-Dichlorcyclopentylchlorid, Hexylchlorid, 1,2-Dichlorhexan, 1,6-Dichlorhexan, 1,2,3,4-Tetrachlorhexan, 1,2,5,6-Tetrachlorhexan, Cyclohexylchlorid, Chlorbenzol, Heptylchlorid, 1,2-Dichlorheptan, 1,2,3,4-Tetrachlorheptan, Cycloheptylchlorid, Octylchlorid, 1,2-Dichloroctan, 1,2,3,4-Tetrachloroctan und Cyclooctylchlorid.

Bevorzugte Lösungsmittel für die erfindungsgemäße Umsetzung von Alkenen mit einer 3 oder 4 C-Atome enthaltenden Percarbonsäure sind von den chlorierten Kohlenwasserstoffen Methylenchlorid, Chloroform, Tetrachlorkohlenstoff und 1,2-Dichlorpropan, von den aromatischen Kohlenwasserstoffen Benzol, Nitrobenzol, Toluol und Chlorbenzol, von den aliphatischen und cycloaliphatischen Kohlenwasserstoffen 2-Ethyl-hexan, Cyclohexan und Methylcyclopentan und von den sauerstoffhaltigen Kohlenwasserstoffen Tetrahydrofuran, Propionsäureethylester und Benzoesäureethylester.

Besonders bevorzugte Lösungsmittel für die erfindungsgemäße Umsetzung von Alkenen mit einer 3 oder 4 C-Atome enthaltenden Percarbonsäure sind von den chlorierten Kohlenwasserstoffen 1,2-Dichlorpropan und Tetrachlorkohlenstoff, von den aromatischen Kohlenwasserstoffen Benzol und Chlorbenzol, von den aliphatischen und cycloaliphatischen Kohlenwasserstoffen Cyclohexan und von den sauerstoffhaltigen Kohlenwasserstoffen Propionsäureethylester und Benzoesäureethylester.

Für die erfindungsgemäße Umsetzung von Alkenen mit einer 3 oder 4 C-Atome enthaltenden Percarbonsäure können auch Gemische der verschiedenen oben angegebenen organischen Lösungsmittel verwendet werden.

Erfindungsgemäß verwendbare Percarbonsäuren sind beispielsweise Perpropionsäure, Perbuttersäure und Perisobuttersäure. Bevorzugt verwendet werden Perpropionsäure und Perisobuttersäure. Besonders bevorzugt ist Perpropionsäure. Diese Percarbonsäuren, gelöst in einem der genannten organischen Lösungsmittel, können z. B. nach dem in der DE-OS 2 262 970 beschriebenen Verfahren hergestellt werden, bei dem wäßriges Wasserstoffperoxid mit der entsprechenden Carbonsäure in Gegenwart von Schwefelsäure umgesetzt und anschließend die entstandene Percarbonsäure mit dem organischen Lösungsmittel aus dem Reaktionsgemisch extrahiert wird. Gegebenenfalls kann die so erhaltene Percarbonsäurelösung in dem organischen Lösungsmittel noch weiter gereinigt werden, insbesondere um den Gehalt an Wasser, Wasserstoffperoxid und Schwefelsäure zu erniedrigen.

Die Lösungen, die organisches Lösungsmittel und Percarbonsäure enthalten, können beispielsweise 10 bis 30 Gew.-% der jeweiligen Percarbonsäure, bezogen auf die Lösung, enthalten. Die Alkene können als solche oder ebenfalls gelöst in einem oder mehreren der vorstehend genannten Lösungsmittel eingesetzt werden, wobei beliebig konzentrierte Lösungen der Alkene zum Einsatz gelangen können. Vorzugsweise werden die Alkene als solche eingesetzt und organische Lösungsmittel nur in Form der Percarbonsäurelösung zugegeben.

Das Molverhältnis von eingesetztem Alken zu eingesetzter Percarbonsäure beträgt erfindungsgemäß 0,9 : 1 bis 5 : 1. Bevorzugt ist ein Molverhältnis von 1,0 : 1 bis 4 : 1. Besonders bevorzugt werden pro Mol Percarbonsäure 1,5 bis 3 Mol Alken eingesetzt.

Der Wassergehalt der verwendeten Percarbonsäurelösung soll im allgemeinen möglichst niedrig sein. Wassermengen bis 5 Gew.-% in der Percarbonsäurelösung sind im allgemeinen nicht störend. Insbesondere geeignet sind beispielsweise Percarbonsäurelösungen mit einem Wassergehalt von bis zu 2 Gew.-%. Vorzugsweise verwendet man Percarbonsäurelösungen, die weniger als 1 Gew.-% Wasser enthalten. Besonders bevorzugt ist ein Wassergehalt von weniger als 0,1 Gew.-%.

Der Wasserstoffperoxidgehalt der verwendeten Percarbonsäurelösung soll im allgemeinen ebenfalls möglichst niedrig sein. Er kann bis zu 0,5 Gew.-%, bezogen auf die Percarbonsäurelösung, betragen. Vorteilhaft arbeitet man bei einem Gehalt von weniger als 0,35 Gew.-%. Besonders vorteilhaft ist es. Die Umsetzung mit einer Percarbonsäurelösung durchzuführen, die einen Wasserstoffperoxidgehalt unterhalb 0,2 Gew.-% aufweist.

Der Mineralsäuregehalt der verwendeten Percarbonsäurelösung soll auch möglichst niedrig sein und unterhalb 50 ppm liegen. Besonders vorteilhaft ist ein Mineralsäuregehalt von weniger als 10 ppm.

Die erfindungsgemäße Umsetzung wird in dem Temperaturbereich von 40 bis 90° C durchgeführt. Bevorzugt arbeitet man bei 45 bis 80° C, besonders bevorzugt bei 50 bis 75° C.

Die erfindungsgemäße Umsetzung kann bei den verschiedenen Drucken durchgeführt werden. Im allgemeinen arbeitet man bei Normaldruck. Das Verfahren kann jedoch auch bei Unter- oder Überdruck durchgeführt werden.

Die Durchführung der erfindungsgemäßen Umsetzung kann diskontinuierlich oder kontinuierlich in den für Umsetzungen dieser Art üblichen Vorrichtungen, wie Rührwerkskesseln, Siedereaktoren, Röhrenreaktoren, Schlaufenreaktoren oder Schleifenreaktoren, erfolgen.

Als Werkstoffe für die Reaktionsapparate zur Durchführung der erfindungsgemäßen Umsetzung können beispielsweise Glas, Edelstähle oder emailliertes Material verwendet werden.

Schwermetallionen im Reaktionsgemisch katalysieren die Zersetzung von Percarbonsäuren. Es ist deshalb vorteilhaft, der Percarbonsäurelösung Substanzen zuzusetzen, die Schwermetallionen durch Komplexbildung inaktivieren können. Substanzen solcher Art sind beispielsweise Gluconsäure, Ethylendiamintetraessigsäure, Natriumsilikat, Natriumpyrophosphat, Natriumhexametaphosphat, Dinatriumdimethylpyrophosphat oder $Na_5$ (2-Ethyl-hexyl)$_5$($P_3O_{10}$)$_2$ (vgl. DE-AS 1 056 596, Spalte 4, Zeilen 60 ff).

Die Reaktionswärme kann auf beliebige Weise abgeführt werden, beispielsweise durch innen- oder außenliegende Kühler. Zur Ableitung der Reaktionswärme kann die Umsetzung auch unter Rückfluß (z. B. in Siedereaktoren) durchgeführt werden.

Das Alken und die Percarbonsäurelösung können auf beliebige Weise zusammengebracht werden. Beispielsweise kann man beide Komponenten gleichzeitig oder nacheinander in beliebiger Reihenfolge in das Reaktionsgefäß einbringen. Bei diskontinuierlicher Arbeitsweise wird vorzugsweise das Alken vorgelegt und dann die Percarbonsäurelösung zugegeben. Die Reaktionstemperatur kann dabei vor oder nach der Zugabe der Percarbonsäurelösung eingestellt werden. Bei kontinuierlicher Arbeitsweise kann man die beiden Komponenten gemeinsam oder getrennt dem Reaktor zuführen. Bei Verwendung mehrerer Reaktoren, die beispielsweise als Kaskade hintereinander geschaltet sein können, kann es vorteilhaft sein, das Alken nur in den ersten Reaktor einzubringen. Man kann die Alkenzugabe jedoch auch auf mehrere Reaktoren verteilen.

Die erfindungsgemäße Umsetzung einsetzbare Alkene sind beispielsweise α-Olefine mit 7 bis 20 C-Atomen, wie Hepten-1, Octen-1, Nonen-1, Decen-1, Undecen-1, Dodecen-1, Tridecen-1, Tetradecen-1, Pentadecen-1, Hexadecen-1, Heptadecen-1, Octadecen-1, Nonadecen-1, und Eikosen-1. Im allgemeinen sind von diesen Alkenen diejenigen, die eine gerade Anzahl von Kohlenstoffatomen enthalten, besser zugänglich. Vorzugsweise wird deshalb Octen-, Decen-1, Dodecen-1, Tetradecen-1, Hexadecen-1, Octadecen-1 oder Eikosen-1 eingesetzt.

Im Rahmen des erfindungsgemäßen Verfahrens kann jedes der einsetzbaren Alkene mit jeder der einsetzbaren Percarbonsäuren umgesetzt werden. Außer dem grundsätzlich bevorzugten Einsatz von Perpropionsäure bestehen keine besonderen Vorzugskombinationen von bestimmten Alkenen mit bestimmten Percarbonsäuren.

Die nach Durchführung der erfindungsgemäßen Umsetzung erhaltenen Reaktionsgemische enthalten stets das verwendete organische Lösungsmittel, die aus der eingestetzten Percarbonsäure entstandene Carbonsäure und das gebildete Alkyloxiran, sowie gegebenenfalls geringe Mengen von hochsiedenden Nebenprodukten. Wenn Alken im molaren Überschuß, bezogen auf eingesetzte Percarbonsäure, eingesetzt wurde, beispielsweise mehr als 1 Mol Alken pro Mol Percarbonsäure, so enthält das Reaktionsgemisch im allgemeinen zusätzlich unumgesetztes Alken. Wenn Alken im molaren Unterschuß, bezogen auf eingesetzte Percarbonsäure, eingesetzt wurde, beispielsweise 0,9 bis 1 Mol Alken pro Mol Percarbonsäure, so enthält das Reaktionsgemisch im allgemeinen kein Alken, kann daher aber geringe Mengen unumgesetzter Percarbonsäure enthalten. Diese geringen Mengen Percarbonsäure stören, falls sie vorhanden sind, die erfindungsgemäße Aufarbeitung nicht. Die Percarbonsäure fällt dann bei der Aufarbeitung entweder zusammen mit der Carbonsäure an und/oder zersetzt sich in gefahrloser Weise, ohne daß sicherheitstechnische Probleme auftreten.

Die Aufarbeitung der nach der erfindungsgemäßen Umsetzung vorliegenden Reaktionsgemische erfolgt, gegebenenfalls nach einer Extraktion mit Wasser, durch Auftrennen der Komponenten durch Destillation. Die Extraktion mit Wasser ist durchzuführen, wenn das eingesetzte Alken oder das daraus entstehende Epoxid ähnliche Siedepunkte besitzt wie die aus der eingesetzten Percaronbsäure entstehende Carbonsäure. Sie ist weiterhin vorteilhaft, wenn Alkene der Formel

$$CH_3\text{-}(CH_2)_n\text{-}CH=CH_2,$$

worin

n für eine ganze Zahl von 4 bis 6 steht,

mit Perpropionsäure umgesetzt werden.

Die Extraktion mit Wasser ist außerdem dann vorteilhaft, wenn das Reaktionsgemisch kein oder nur geringe Mengen nicht umgesetztes Alken enthält, beispielsweise dann, wenn die Umsetzung des Alkens mit der Percarbonsäure bei einem Molverhältnis von Alken zu Percarbonsäure im Bereich von 0,9 : 1 bis 1,5 : 1 durchgeführt wurde.

Die direkte Destillation des Reaktionsgemisches zur Auftrennung in seine Komponenten (ohne vorgeschaltete Extraktion mit Wasser) kann beispielsweise, wenn das Olefin höher als die Carbonsäure siedet, gemäß einer der folgenden Varianten durchgeführt werden:

Variante 1

(diskontinuierlich)

Hierbei werden die einzelnen Komponenten in der Reihenfolge ihrer Siedepunkte fraktioniert destilliert, wobei nacheinander Fraktionen erhalten werden, die im wesentlichen das verwendete Lösungsmittel, die aus der Percarbonsäure entstandene Carbonsäure, nichtumgesetztes Alken und das gebildete Alkyoxiran enthalten. Das abgetrennte, nicht-umgesetzte Alken kann mit soviel Frisch-Alken versetzt werden, wie bei der Umsetzung von Alken mit der Percarbonsäure verbraucht worden ist, und

in die Umsetzung mit Percarbonsäure zurückgeführt werden. Das abgetrennte Lösungsmittel und die abgetrennte Carbonsäure können zur Herstellung der erfindungsgemäß einzusetzenden Percarbonsäurelösung eingesetzt werden.

Variante 2

(kontinuierlich)

Das Reaktionsgemisch wird in eine erste Destillationseinheit gegeben, in der das Lösungsmittel als Kopfprodukt gewonnen wird. Das Sumpfprodukt wird in eine zweite Destillationseinheit gegeben, in der die aus der Percarbonsäure entstandene Carbonsäure als Kopfprodukt gewonnen wird. Das Sumpfprodukt wird in eine dritte Destillationseinheit gegeben, in der nicht-umgesetztes Alken als Kopfprodukt gewonnen wird. Nach Hinzufügen von soviel Frisch-Alken, wie bei der Umsetzung von Alken mit der Percarbonsäure verbraucht worden ist, kann das abgetrennte, nicht umgesetzte Alken in die Umsetzung mit Percarbonsäure zurückgeführt werden. Das Sumpfprodukt der dritten Destillationseinheit wird in eine vierte Destillationseinheit gegeben, in der als Kopfprodukt das gebildete Alkyloxiran und als Sumpfprodukt geringe Mengen hochsiedender Nebenprodukte erhalten werden. Auch hierbei kann das abgetrennte Lösungsmittel und die abgetrennte Carbonsäure zur Herstellung der erfindungsgemäß einzusetzenden Percarbonsäurelösung eingesetzt werden.

Variante 3

(kontinuierlich)

Das Reaktionsgemisch wird einer ersten Destillationseinheit zugeführt, in der als Kopfprodukt ein Gemisch enthaltend das Lösungsmittel und die aus der Percarbonsäure entstandene Carbonsäure und als Sumpfprodukt ein Gemisch enthaltend unumgesetztes Alken und gebildetes Alkyoxiran gewonnen wird. Das Sumpfprodukt der ersten Destillationseinheit wird einer zweiten Destillationseinheit zugeführt, in der das unumgesetzte Alken als Kopfprodukt und das gebildete Alkyloxiran als Sumpfprodukt erhalten wird. Das Sumpfprodukt der zweiten Destillationseinheit wird einer dritten Destillationseinheit zugeführt, in der das gebildete Alkyloxiran als Kopfprodukt und geringe Mengen hochsiedender Nebenprodukte als Sumpfprodukt erhalten werden. Das Kopfprodukt der ersten Destillationseinheit wird einer vierten Destillationseinheit zugeführt, in der das Lösungsmittel als Kopfprodukt und die aus der Percarbonsäure entstandenen Carbonsäure als Sumpfprodukt erhalten wird. Das Sumpfprodukt der vierten Destillationseinheit kann gegebenenfalls in einer fünften Destillationseinheit gereinigt werden. Das abgetrennte, nicht-umgesetzte Alken kann nach Zugabe einer Menge Frisch-Alken, die der in der Umsetzung mit der Percarbonsäure verbrauchten Menge Alken entspricht, in die Umsetzung mit der Percarbonsäure zurückgeführt werden. Das abgetrennte Lösungsmittel und die abgetrennte und gegebenenfalls gereinigte Carbonsäure können zur Herstellung der erfindungsgemäß einzusetzenden Percarbonsäurelösung eingesetzt werden.

Variante 4

(kontinuierlich)

Das Reaktionsgemisch wird einer ersten Destillationseinheit zugeführt, in der als Kopfprodukt ein Gemisch enthaltend das Lösungsmittel, die aus der Percarbonsäure entstandenen Carbonsäure und ganz oder teilweise das nicht-umgesetzte Alken und ein Sumpfprodukt, enthaltend das gebildete Oxiran und gegebenenfalls restliches unumgesetztes Alken, gewonnen wird. Wenn das Sumpfprodukt der ersten Destillationseinheit Alken enthält, wird dieses Sumpfprodukt einer zweiten Destillationseinheit zugeführt, in der als Kopfprodukt das nicht-umgesetzte Alken gewonnen wird. Wenn das Sumpfprodukt der ersten Destillationseinheit kein Alken enthält, entfällt die zweite Destillationseinheit. In einer dritten Destillationseinheit wird entweder Alken-freies Sumpfprodukt der ersten Destillationseinheit oder das Sumpfprodukt der zweiten Destillationseinheit aufgetrennt in das gebildete Alkyloxiran als Kopfprodukt und geringe Mengen hochsiedender Nebenprodukte als Sumpfprodukt. In einer vierten Destillationseinheit wird das Kopfprodukt der ersten Destillationseinheit aufgetrennt in ein Kopfprodukt, welches das Lösungsmittel enthält, und in ein Sumpfprodukt, das die Carbonsäure und nicht-umgesetztes Alken enthält. In einer fünften Destillationseinheit wird das Sumpfprodukt der vierten Destillationseinheit getrennt, wobei die Carbonsäure als Kopfprodukt und nicht-umgesetztes Alken als Sumpfprpodukt erhalten wird. Sofern die zweite Destillationseinheit vorhanden ist, wird das Sumpfprodukt der fünften Destillationseinheit in die zweite Destillationseinheit zurückgeführt. Falls die zweite Destillationseinheit nicht vorhanden ist, wird das gesamte unumgesetzte Alken als Sumpfprodukt der fünften Destillationseinheit erhalten und kann gegebenenfalls durch nochmalige Verdampfung oder Destillation gereinigt werden. Das entweder als Kopfprodukt der zweiten oder als Sumpfprodukt der fünften Destillationseinheit anfallende und gegebenenfalls weiter gereinigte nichtumgesetzte Alken kann mit soviel Frisch-Alken versetzt werden, wie in der Umsetzung mit der Percarbonsäure verbraucht wurde, und in die Umsetzung mit der Percarbonsäure zurückgeführt werden. Das abgetrennte Lösungsmittel und die abgetrennte Carbonsäure kann zur Herstellung der erfindungsgemäß einzusetzenden Percarbonsäurelösung eingesetzt werden.

Bei allen 4 beispielhaft beschriebenen Varianten der direkten Destillation des Reaktionsgemisches zur Auftrennung in seine Komponente ist es möglich und bevorzugt, dem abgetrennten nicht-umgesetzten Alken soviel Frisch-Alken zuzusetzen, wie in der Umsetzung mit der Percarbonsäure verbraucht worden ist und es in die Umsetzung mit Percarbonsäure zurückzuführen, sowie das Lösungsmittel und die Carbonsäure in gereinigter Form abzutrennen und beispielsweise zur Herstellung der erfindungsgemäßen einzusetzenden Percarbonsäurelösung zu verwenden.

Die destillative Auftrennung des Reaktionsgemisches in seine Komponenten mit vorgeschalteter Extraktion mit Wasser kann beispielsweise so durchgeführt werden, daß man das Reaktionsgemisch mit der ein- bis dreifachen Gewichtsmenge Wasser extrahiert, wobei man eine Carbonsäure enthaltende wäßrige Phase und ein weitgehend von Carbonsäure freies Reaktionsgemisch erhält, das dann destillativ in seine Komponenten aufgetrennt wird. Man kann diese Destillation beispielsweise diskontinuierlich durchführen und die einzelnen Bestandteile des mit Wasser extrahierten Reaktionsgemisches durch Fraktionierung in der Reihenfolge ihrer Siedepunkte abtrennen, d. h. im allgemeinen in der Reihenfolge: Lösungsmittel, gegebenenfalls unumgesetztes Alken, gebildetes Alkyloxiran. Man kann diese Destillation auch kontinuierlich durchführen, indem man beispielsweise in einer ersten Destillationseinheit die am niedrigsten siedende Komponente, im allgemeinen das Lösungsmittel, als Kopfprodukt abtrennt, das Sumpfprodukt der ersten Destillationseinheit in eine zweite Destillationseinheit einleitet, dort als Kopfprodukt die nächsthöhersiedende Komponente abtrennt und das Sumpfprodukt der zweiten Destillationseinheit gegebenenfalls in eine dritte Destillationseinheit einleitet und dort gegebenenfalls die nächsthöhersiedende Komponente als Kopfprodukt abtrennt.

Wenn das aufzuarbeitende Reaktionsgemisch kein nichtumgesetztes Alken enthält, sind für die kontinuierliche Destillation im allgemeinen zwei Destillationseinheiten ausreichend, wobei man in der ersten Destillationseinheit das Lösungsmittel als Kopfprodukt und in der zweiten Destillationseinheit als Kopfprodukt das gebildete Alkyloxiran und als Sumpfprodukt geringe Mengen hochsiedender Nebenprodukte abtrennt. Wenn das aufzuarbeitende Reaktionsgemisch nicht-umgesetztes Alken enthält, sind für die kontinuierliche Destillation im allgemeinen drei Destillationseinheiten erforderlich, wobei man im allgemeinen in der ersten Destillationseinheit das Lösungsmittel als Kopfprodukt, in der zweiten Destillationseinheit das nicht-umgesetzte Alken als Kopfprodukt und in der dritten Destillationseinheit das gebildete Alkyloxiran als Kopfprodukt und geringe Mengen hochsiedender Nebenprodukte als Sumpfprodukt abtrennt. Man kann jedoch auch so verfahren, daß man in der ersten Destillationseinheit das Lösungsmittel und das nicht-umgesetzte Alken gemeinsam als Kopfprodukt abtrennt, aus dem Sumpfprodukt der ersten Destillationseinheit in einer zweiten Destillationseinheit das gebildete Alkyoxiran als Kopfprodukt und geringe Mengen hochsiedender Verunreinigungen als Sumpfprodukt abtrennt und aus dem Kopfprodukt der ersten Destillationseinheit in einer dritten Destillationseinheit das Lösungsmittel als Kopfprodukt und das nicht-umgesetzte Alken als Sumpfprodukt abtrennt.

Wenn das aufzuarbeitende Reaktionsgemisch nicht-umgesetztes Alken enthält und folglich bei der Destillation des mit Wasser extrahierten Reaktionsgemisches nicht-umgesetztes Alken anfällt, so ist es bevorzugt, diesem abgetrennten nicht-umgesetzten Alken soviel Frisch-Alken zuzufügen, wie in der Umsetzung mit Percarbonsäure gebraucht worden ist und es in die erfindungsgemäße Umsetzung mit der Percarbonsäure zurückzuführen.

Die wäßrige Phase aus der Extraktion des Reaktionsgemisches mit Wasser wird vorzugsweise in einer zweiten Extraktion mit einem organischen Lösungsmittel extrahiert. Als organische Lösungsmittel kommen hierfür die verschiedensten Lösungsmittel in Frage, die mit Wasser eine Mischungslücke bilden. Beispielsweise können diejenigen Lösungsmittel verwendet werden, die vorstehend als Lösungsmittel für die erfindungsgemäße Umsetzung beschrieben sind. Vorzugsweise werden die dort angegebenen Carbonsäurealkylester, chlorierten Kohlenwasserstoffe oder aromatischen Kohlenwasserstoffe eingesetzt, besonders bevorzugt die dort beschriebenen Carbonsäurealkylester. Ganz besonders bevorzugt sind Essigsäurebutylester, Propionsäureethylester, Buttersäurepropylester und Isobuttersäurepropylester.

Bei der zweiten Extraktion kann beispielsweise die 1- bis 3fache Gewichtsmenge Lösungsmittel, bezogen auf die zu extrahierende wäßrige Phase, eingesetzt werden.

Sowohl die erste, als auch die zweite Extraktion können beispielsweise bei Temperaturen im Bereich 10 bis 60°C durchgeführt werden. Bevorzugt wird bei Normaldruck und Raumtemperatur (18 bis 25°C) gearbeitet.

Bei der zweiten Extraktion fällt eine wäßrige Phase an, die nur noch wenig Carbonsäure enthält und eine organische Phase, die als wesentliche Bestandteile das verwendete Lösungsmittel und die Carbonsäure enthält.

Besonders bevorzugt ist es, die wäßrige Phase der zweiten Extraktion in die Extraktion des Reaktionsgemisches zurückzuführen und aus der organischen Phase der zweiten Extraktion durch Destillation des Lösungsmittels und die Carbonsäure abzutrennen und das Lösungsmittel wieder in der zweiten Extraktion zu verwenden. Die Carbonsäure wird vorzugsweise zur Herstellung der für die erfindungsgemäße Umsetzung benötigten Percarbonsäurelösung eingesetzt. Für die destillative Abtrennung von Lösungsmittel und Carbonsäure aus der organischen Phase der zweiten Extraktion können an sich für solche Abtrennungen bekannte Arbeitsweisen angewendet werden.

Bei allen beschriebenen Destillationen des extrahierten oder nicht-extrahierten Reaktionsgemisches werden vorzugsweise Verdampfer eingesetzt, die kurze Verweilzeiten und eine Verdampfung mit geringer thermischer Belastung gestatten, beispielsweise Fallstromverdampfer oder Dünnschichtverdampfer. Diese Destillationen werden ferner vorzugsweise unter vermindertem Druck durchgeführt, insbesondere in denjenigen Teilen, in denen relativ hochsiedende Komponenten, beispielsweise Carbonsäure, nicht-umgesetztes Alken oder gebildetes Alkyloxiran als Kopfprodukte anfallen. Beispielsweise können dabei Drucke im Bereich 0,1 bis 500 mbar angewendet werden. Solche Teile, in denen das Lösungsmittel als Kopfprodukt anfällt, können ohne Probleme auch bei Normaldruck betrieben werden oder auch bei nur mäßig erniedrigten Drucken, beispielsweise bei 20 bis 600 mbar.

Es kann vorteilhaft sein, dem Reaktionsgemisch vor oder während der Aufarbeitung, insbesondere bei der destillativen Aufarbeitung, Stabilisatoren zuzusetzen, welche die Bildung von Hochsiedern und Polymerisaten verhindern.

Das erfindungsgemäße Verfahren hat eine Reihe von überraschenden Vorteilen. So ist es mit diesem Verfahren möglich, nach der sogenannten Prileschajew-Reaktion in technischem Maßstab Alkyloxirane in hohen Ausbeuten, in hoher Reinheit, wirtschaftlich und ohne Bildung umweltbelastender Salzabwässer herzustellen. Auch bei isothermer Temperaturführung der Umsetzung von Alken mit Percarbonsäure gelingt es erfindungsgemäß, Alkyloxirane in hohen Ausbeuten und Selektivitäten zu erhalten, obwohl gemäß der DE-AS 1 230 005 bei derartigen Reaktionen ein kompliziertes Temperaturführungsprogramm erforderlich ist. Erfindungsgemäß sind nur kurze Reaktionszeiten erforderlich, die für eine technische Anwendung sehr vorteilhaft sind. Bisher wurde die destillative Trennung Alken/Alkyloxiran vermieden, die relativ hohe Temperaturen erfordert, indem man Percarbonsäure im Überschuß einsetzte und somit immer ein Alken-freies Reaktionsgemisch erhielt, in dem dann allerdings in unwirtschaftlicher und sicherheitstechnisch nicht unbedenklicher Weise überschüssige Percarbonsäure zu zerstören war. Erfindungsgemäß kann man auch mit überschüssigem Alken arbeiten, das zurückgewonnen und wiederverwendet werden kann. Weiterhin ist es überraschend, daß erfindungsgemäß auch ein Carbonsäure und Alkyloxiran enthaltendes Gemisch destillativ aufgearbeitet werden kann, denn der Fachmann erwartet dabei Nebenreaktionen (siehe S. N. Lewis in R. L. Augustin a. a. O.). Bisher wurde stets Säure aus dem Epoxid durch Waschen mit Wasser entfernt, was jedoch zu umweltbelastenden Abwässern führte. Schließlich ist es erfindungsgemäß möglich, ohne umweltbelastende Abwässer zu erhalten, alle bei der Aufarbeitung anfallenden Ströme, soweit es sich nicht um das hergestellte Alkyloxiran und geringe Mengen hochsiedender Nebenprodukte handelt, wieder zu verwenden.

## Beispiele

### Beispiel 1

896 g (4 Mol) Hexadecen-1 wurden in einem 4 l Dreihalskolben, der mit einem KPG-Rührer, einem Rückflußkühler und einem Tropftrichter versehen war, vorgelegt und auf 60° C erwärmt. Unter Rühren tropft man 900 g (2 Mol) einer 20gew.-%igen Lösung von Perpropionsäure in Benzol, die weniger als 0,1 Gew.-% Wasser, weniger als 0,2 Gew.-% Wasserstoffperoxid und weniger als 10 ppm Schwefelsäure enthielt, so schnell zu, daß die Temperatur bei 60° C gehalten werden konnte. Anschließend ließ man bei 60° C noch 2 Stunden nachrühren. Dann zeigte die titrimetrische Analyse nach der Natriumthiosulfat-Methode einen Percarbonsäure-Umsatz von 99% an. Gemäß gaschromatographischer Analyse ist das Tetradecyloxiran mit einer Selektivität von 94,6%, bezogen auf umgesetztes Hexadecen-1, gebildet worden.

Zur Aufarbeitung des Reaktionsgemisches wurde der Tropftrichter durch eine 30 cm Füllkörperkolonne ersetzt, die mit 4 mm Raschigringen aus Glas gefüllt war, und bei einem Druck von 195 mbar wurde Benzol bei einer Kopftemperatur von 35° C abdestilliert. Anschließend wurde das Vakuum auf 20 mbar erniedrigt und Propionsäure wurde bei einer Kopftemperatur von 46° C als Kopfprodukt erhalten. Bei einem Druck von 7 mbar wurde dann das überschüssige Hexadecen-1 bei einer Kopftemperatur von 128° C abdestilliert. Nach Zusatz der in der Reaktion verbrauchten Menge Hexadecen-1 wurde das so abgetrennte Hexadecen-1 im nächsten Reaktionsansatz wieder verwendet. In der Destillationskolonne wurde anschließend der Druck auf 0,6 mbar erniedrigt, wobei Tetradecyloxiran bei einer Kopftemperatur von 131° C in reiner Form erhalten wurde.

Beispiel 2

In einer dreistufigen Kesselkaskade, die aus 3 Glasgefäßen mit jeweils 1 l Inhalt bestand, wurden über getrennte Leitungen in den ersten Kessel stündlich 392 g (2 Mol) Tetradecen-1 und 437 g (1 Mol) einer 20,6%igen Lösung von Perpropionsäure in Benzol dosiert, welche die gleichen Spezifikationen aufwies wie in Beispiel 1 angegeben. Bei einer Reaktionstemperatur von 60°C resultierte im dritten Kessel der Kaskade ein Percarbonsäureumsatz von 99,4%. Dodecyloxiran wurde mit einer Selektivität von 95,4% gebildet, bezogen auf umgesetztes Olefin.

Das die Kaskade verlassende Reaktionsgemisch wurde in eine Destillationskolonne geleitet, in der bei einem Druck von 200 mbar Benzol und Propionsäure als gemeinsames Kopfprodukt erhalten wurden. Dieses Kopfprodukt wurde anschließend in einer weiteren Destillationskolonne getrennt. Das so erhaltene Benzol und die so erhaltene Propionsäure wurden wieder zur Herstellung der Perpropionsäurelösung verwendet. Aus dem Sumpfprodukt der ersten Kolonne wurde in einer weiteren Destillationskolonne bei einem Druck von 20 mbar und einer Kopftemperatur von 125°C das nicht-umgesetzte Tetradecen-1 erhalten. Es wurde nach Zusatz der Menge an Tetradecen-1, die durch die Reaktion verbraucht worden war, in den ersten Kessel der Reaktionskaskade zur erneuten Umsetzung mit Perpropionsäurelösung zurückgeführt. Aus dem Sumpfprodukt dieser Kolonne wurde anschließend in einer weiteren Destillationskolonne Dodecyloxiran bei einem Druck von 8 mbar und einer Kopftemperatur von 134°C in einer Menge von 191 g pro Stunde als Kopfprodukt erhalten.

Beispiel 3

504 g (3 Mol) Dodecen-1 und 459,2 g einer 19,6gew.-%igen Lösung von Perpropionsäure in Benzol, welche die gleiche Spezifikation hatte wie in Beispiel 1 angegeben, wurden bei einer Reaktionstemperatur von 50°C stündlich in den ersten Kessel einer vierstufigen Kesselkaskade eingeleitet, die aus Glasgefäßen mit einem Gesamtvolumen von 3 l bestand. Dabei wurde im vierten Kessel ein Percarbonsäureumsatz von 99,6% erhalten. Decyloxiran ist nach gaschromatographischer Analyse mit einer Selektivität von 96,1%, bezogen auf umgesetztes Olefin, gebildet worden.

Das die Kaskade verlassende Reaktionsgemisch wurde in einer ersten Destillationskolonne bei einem Druck von 200 mbar in ein Kopfprodukt, bestehend aus Benzol, Propionsäure und etwa 40% des nicht-umgesetzten Dodecen-1 und in ein Sumpfprodukt aufgetrennt, in dem der Rest nicht-umgesetztes Dodecen-1, Decyloxiran und kleine Mengen an höhersiedenden Produkten enthalten waren. In einer zweiten Destillationskolonne wurde aus dem Kopfprodukt der ersten Kolonne Benzol bei einem Druck von 260 mbar und einer Kopftemperatur von 43°C abdestilliert. Das Sumpfgemisch der zweiten Kolonne wurde in einer dritten Kolonne bei einem Druck von 66 mbar und einer Kopftemperatur von 71°C in ein Kopfprodukt, bestehend aus Propionsäure und in ein Sumpfprodukt, bestehend aus Dodecen-1, aufgetrennt. Das Sumpfprodukt dieser Kolonne wurde mit dem Sumpfprodukt der ersten Kolonne vereinigt und in einer vierten Kolonne wurde bei einem Druck von 13 mbar und einer Kopftemperatur von 89°C das nicht-umgesetzte Dodecen-1 erhalten, das nach Zugabe der Menge an Frisch-Dodecen-1, die durch die Reaktion verbraucht worden war, in den ersten Kessel der Reaktionskaskade zurückgeführt wurde. Aus dem Sumpfprodukt der vierten Kolonne wurde Decyloxiran in einer fünften Kolonne bei einem Druck von 2 mbar und einer Kopftemperatur von 84°C in einer Menge von 169 g pro Stunde als Kopfprodukt erhalten.

Beispiel 4

In entsprechender Weise wie in Beispiel 2 beschrieben wurden 280 g (2 Mol) Decen-1 bei einer Reaktionstemperatur von 60°C mit 22gew.-%iger benzolischer Perpropionsäurelösung bei einem Molverhältnis von Decen-1 zu Perpropionsäure von 2 : 1 umgesetzt. Die verwendete Perpropionsäurelösung wies die gleiche Spezifikation auf wie in Beispiel 1 angegeben. Im dritten Kessel der Kaskade resultierte ein Percarbonsäure-Umsatz bei einer mittleren Verweilzeit von 3 Stunden von 99%. Octyloxiran wurde mit einer Selektivität von 95%, bezogen auf umgesetztes Decen-1, gebildet.

Das die Kaskade verlassende Reaktionsgemisch wurde in einer ersten Destillationskolonne bei einem Druck von 200 mbar in ein Kopfprodukt, bestehend aus Benzol, Propionsäure und dem nicht-umgesetzten Decen-1 und in ein Sumpfprodukt aufgetrennt, in dem Octyloxiran und kleine Mengen an höhersiedenden Produkten enthalten waren. Aus dem Kopfprodukt dieser Kolonne wurde in einer zweiten Destillationskolonne Benzol und in einer dritten Destillationskolonne Propionsäure jeweils als Kopfprodukt erhalten. Das in der dritten Destillationskolonne als Sumpfprodukt erhaltene Decen-1 wurde nach Zugabe der Menge an Decen-1, die durch die Reaktion verbraucht worden war, in den ersten Kessel der Reaktionskaskade zurückgeführt. Aus dem Sumpfprodukt der ersten Kolonne wurde in einer vierten Kolonne bei einem Druck von 13 mbar und einer Kopftemperatur von 90°C Octyloxiran als Kopfprodukt in einer Menge von 142 g pro Stunde erhalten.

Beispiel 5

In einer fünfstufigen Kesselkaskade aus Glas mit einem Gesamtvolumen von 5 l wurden stündlich bei einer Reaktionstemperatur von 70°C 450 g einer 20gew.-%igen (1 Mol) Perpropionsäurelösung in Benzol und 126 g (0,9 Mol) Decen-1 über getrennte Leitungen in den ersten Kessel der Kaskade dosiert. Die Percarbonsäurelösung enthielt weniger als 0,1 Gew.-% Wasser, weniger als 0,2 Gew.-% Wasserstoffperoxid und weniger als 10 ppm Mineralsäure. Nach dem fünften Kessel resultierte ein Decen-1-Umsatz von 99,6%. Die gaschromatographische Analyse ergab, daß Octyloxiran in einer Ausbeute von 93% der Theorie erhalten worden ist.

Das die Kaskade verlassende Reaktionsgemisch wurde in einem ersten Extraktor, einem pulsierten Siebbodenextraktor, mit der 1,5fachen Gewichtsmenge Wasser im Gegenstrom extrahiert. Dabei erhielt man eine am Kopf des Extraktors abgezogene organische Phase mit einem Propionsäureanteil von unter 0,1 Gew.-%. Aus diesem Gemisch wurde in einer ersten Destillationskolonne bei einem Druck von 200 mbar und einer Kopftemperatur von 36 bis 37°C Benzol in einer zur Herstellung der eingangs genannten Percarbonsäurelösung geeigneten Reinheit abgetrennt. Aus dem Sumpf dieser Kolonne wurde anschließend bei einem Druck von 13 mbar und einer Kopftemperatur von 90°C pro Stunde 180 g Octyloxiran als Kopfprodukt erhalten. Die am unteren Ende des ersten Extraktors abgezogene wäßrige Phase, welche Propionsäure enthielt, wurde in einem zweiten Extraktor, ebenfalls einem pulsierten Siebbodenextraktor, mit der 1,5fachen Gewichtsmenge Propionsäureethylester im Gegenstrom extrahiert. Das diesen Extraktor verlassende, weitgehend Propionsäure-freie Wasser wurde zur Extraktion des Reaktionsgemisches in den ersten Extraktor zurückgeführt. Die den zweiten Extraktor verlassende organische Phase, welche die Propionsäure und Propionsäureethylester enthielt, wurde in einer Destillationskolonne in ein Kopfprodukt, bestehend aus dem Propionsäureethylester und in ein Sumpfprodukt aufgetrennt, das im wesentlichen aus Propionsäure bestand. Das Kopfprodukt dieser Destillationskolonne wurde in den zweiten Extraktor zur Extraktion der wäßrigen Phase aus dem ersten Extraktor zurückgeführt.

Beispiel 6

151,2 g (0,9 Mol) Dodecen-1 und 450 g einer 20gew.-%igen (1 Mol) Perpropionsäure in Benzol wurden stündlich wie im Beispiel 5 beschrieben umgesetzt. Die Perpropionsäurelösung hatte dieselbe Spezifikation wie in Beispiel 5 angegeben. Es resultierte ein Dodecen-1-Umsatz von 99,3% und Decyloxiran wurde in einer Ausbeute von 93,6% der Theorie gebildet. Die Aufarbeitung des Reaktionsgemisches erfolgte in analoger Weise wie in Beispiel 5 beschrieben. Decyloxiran wurde bei einem Druck von 2 mbar und bei einer Kopftemperatur von 84°C in einer Menge von 165 g pro Stunde erhalten.

Die den ersten Extraktor verlassende wäßrige Phase, welche die Propionsäure enthält, wurde in dem zweiten Extraktor mit Essigsäurebutylester extrahiert. Das dabei erhaltene, weitgehend Propionsäure-freie Wasser wurde zur Extraktion des Reaktionsgemisches in den ersten Extraktor zurückgeführt. Die den zweiten Extraktor verlassende organische Phase, welche die Propionsäure und Essigsäurebutylester enthielt, wurde in einer Destillationskolonne in Essigsäurebutylester als Kopfprodukt und Propionsäure als Sumpfprodukt aufgetrennt. Der so abgetrennte Essigsäurebutylester wurde in den zweiten Extraktor zurückgeführt.

Beispiel 7

In einer dreistufigen Kesselkaskade aus Glas mit einem Volumen von 1 l pro Kessel wurden über getrennte Leitungen in den ersten Kessel der Kaskade stündlich 391 g (2 Mol) Tetradecen-1 und 437 g (1 Mol) einer 20,6%igen Lösung von Perpropionsäure in Benzol zudosiert. Die Spezifikation der eingesetzten Perpropionsäurelösung in Benzol entsprach derjenigen des Beispiels 5. Bei einer Reaktionstemperatur von 60°C resultierte im dritten Kessel der Kaskade ein Persäureumsatz von 99,4%. Dodecyloxiran wurde mit einer Selektivität von 95,4%, bezogen auf umgesetztes Olefin, gebildet.

Das die Kaskade verlassende Reaktionsgemisch wurde in einem ersten Extraktor mit der 1,5fachen Gewichtsmenge an Wasser extrahiert. Die diesen Extraktor verlassende organische Phase, bestehend aus Benzol, nicht-umgesetztem Tetradecen-1, Dodecyloxiran und weniger als 0,1 Gew.-% Propionsäure wurde in einer ersten Destillationskolonne bei 200 mbar und 36 bis 37°C Kopftemperatur in Benzol als Kopfprodukt und in ein Sumpfprodukt aufgetrennt, aus dem in einer zweiten Destillationskolonne bei einem Druck von 20 mbar und einer Kompftemperatur von 125°C Tetradecen-1 erhalten wurde. Nach Zusatz der Menge an Tetradecen-1, die bei der Reaktion verbraucht worden war, wurde das Tetradecen-1 zur erneuten Reaktion mit der Perpropionsäurelösung in Benzol in die Reaktionskaskade zurückgeführt. Aus dem Sumpf der zweiten Destillationskolonne wurde bei einem Druck von 8 mbar und einer Kopftemperatur von 134°C Dodecyloxiran in einer Menge von 193 g pro Stunde durch erneute Destillation erhalten.

Die den ersten Extraktor verlassende wäßrige Phase, welche die Propionsäure enthält, wurde in dem zweiten Extraktor mit Essigsäurebutylester extrahiert. Das dabei erhaltene, weitgehend Propionsäure-freie Wasser wurde zur Extraktion des Reaktionsgemisches in den ersten Extraktor zurückgeführt. Die den zweiten Extraktor verlassende organische Phase, welche die Propionsäure und Essigsäurebutylester enthielt, wurde in einer Destillationskolonne in Essigsäurebutylester als Kopfprodukt und Propionsäure als Sumpfprodukt aufgetrennt. Der so abgetrennte Essigsäurebutylester wurde in den zweiten Extraktor zurückgeführt.

## Patentansprüche

1. Verfahren zur Herstellung von Isolierung von Alkyloxiranen durch Umsetzung von Alkenen mit Percarbonsäuren, wobei anschließend das Reaktionsgemisch, gegebenenfalls nach einer Extraktion mit Wasser, durch Destillation in seine Komponenten aufgetrennt wird, dadurch gekennzeichnet, daß man ein Alken der Formel

$$CH_3\text{-}(CH_2)_n\text{-}CH=CH_2$$

worin

n für eine ganze Zahl von 4 bis 17 steht,

mit einer Lösung einer 3 oder 4 C-Atome enthaltenden Percarbonsäure in einem organischen Lösungsmittel bei einer Temperatur von 40 bis 90°C und einem Molverhältnis von Alken zu Percarbonsäure von 0,9 : 1 bis 5 : 1 umsetzt, wobei die Percarbonsäurelösung unter 5 Gew.-% Wasser, unter 0,5 Gew.-% Wasserstoffperoxid und unter 50 ppm Mineralsäure enthält.

2. Verfahren nach Anspruch 1, dadurch gekennzeichnet, daß man als Lösungsmittel für die Umsetzung ein Lösungsmittel aus der Gruppe der aliphatischen Kohlenwassersoffe, der cycloaliphatischen Kohlenwasserstoffe, der aromatischen Kohlenwasserstoffe, der Ether, der Ester oder der chlorierten Kohlenwasserstoffe einsetzt.

3. Verfahren nach Anspruch 1 und 2, dadurch gekennzeichnet, daß man die Destillation des Reaktionsgemisches, die gegebenenfalls nach einer Extraktion mit Wasser durchgeführt wird, bei Drucken im Bereich von 0,1 bis 500 mbar durchführt.

4. Verfahren nach Anspruch 1 bis 3, dadurch gekennzeichnet, daß man das Reaktionsgemisch mit der ein -bis dreifachen Gewichtsmenge Wasser extrahiert, wobei man eine Carbonsäure enthaltende wäßrige Phase und ein weitgehend von Carbonsäure freies Reaktionsgemisch erhält und letzteres destillativ in seine Komponenten auftrennt.

5. Verfahren nach Anspruch 4, dadurch gekennzeichnet, daß man die wäßrige Phase aus der Extraktion in einer zweiten Extraktion mit der ein- bis dreifachen Gewichtsmenge (bezogen auf die zu extrahierende wäßrige Phase) eines Lösungsmittels, das mit Wasser eine Mischungslücke bildet, extrahiert.

6. Verfahren nach Ansprüchen 4 und 5, dadurch gekennzeichnet, daß man die Destillation der organischen Phase aus der Extraktion des Reaktionsgemisches mit Wasser kontinuierlich durchführt und dabei in einer ersten Destillationseinheit das Lösungsmittel als Kopfprodukt abtrennt, in einer zweiten Destillationseinheit das nicht-umgesetzte Alken als Kopfprodukt abtrennt und in einer dritten Destillationseinheit das gebildete Alkyloxiran als Kopfprodukt abtrennt.

## Claims

1. Process for the preparation and isolation of alkyloxiranes by reacting alkenes with percarboxylic acids, the reaction mixture, if necessary following an extraction with water, being subsequently separated into its components by distillation, characterized in that an alkene of the formula

$$CH_3\text{-}(CH_2)_n\text{-}CH = CH_2$$

wherein

n represents an integer from 4 to 17,

is reacted with a solution of a percarboxylic acid, containing 3 or 4 C atoms, in an organic solvent at a temperature of 40 to 90°C and with a molar ratio of alkene to percarboxylic acid of 0.9 : 1 to 5 : 1, the percarboxylic acid solution containing less than 5% by weight of water, less than 0.5% by weight of hydrogen peroxide and less than 50 ppm of mineral acid.

2. Process according to claim 1, characterized in that a solvent from the group of the aliphatic hydrocarbons, the cycloaliphatic hydrocarbons, the aromatic hydrocarbons, the ethers, the esters or the chlorinated hydrocarbons is employed as the solvent for the reaction.

3. Process according to claim 1 and 2, characterized in that the distillation of the reaction mixture, which is carried out optionally after an extraction with water, is effected at pressures in the range of 0.1 to 500 mbar.

4. Process according to claim 1 to 3, characterized in that the reaction mixture is extracted with an equal to three-fold amount by weight of water, an aqueous phase containing carboxylic acid and a reaction mixture substantially free from carboxylic acid being obtained and the latter being separated into its components by distillation.

5. Process according to claim 4, characterized in that the aqueous phase from the extraction is extracted, in a second extraction, with an equal to three-fold amount by weight (relative to the aqueous phase to be extracted) of a solvent which forms a miscibility gap with water.

6. Process according to claims 4 and 5, characterized in that the distillation of the organic phase from the extraction of the reaction mixture with water is carried out continuously and, in doing so, the solvent is separated off as the top product in a first distillation unit, the unconverted alkene is separated off as the top product in a second distillation unit and the alkyloxirane formed is separated off as the top product in a third distillation unit.

**Revendications**

1. Procédé de préparation et d'isolation d'alkyl-oxirannes par réaction d'alcènes avec des acides percarboxyliques, le mélange réactionnel étant ensuite séparé en ses composants par distillation, éventuellement après une extraction avec de l'eau, caractérisé en ce qu'on fait réagir un alcène de formule:

$$CH_3-(CH_2)_n-CH = CH_2$$

où

n représente un nombre entier de 4 à 17,

avec une solution d'un acide percarboxylique contenant 3 ou 4 atomes de carbone, dans un solvant organique, à une température se situant dans l'intervalle de 40 à 90° C et avec un rapport molaire de 0,9 : 1 à 5 : 1 entre l'alcène et l'acide percarboxylique, la solution d'acide percarboxylique contenant moins de 5% en poids d'eau, moins de 0,5% en poids de peroxyde d'hydrogène et moins de 50 parties par million d'acide minéral.

2. Procédé suivant la revendication 1, caractérisé en ce que, comme solvant pour la réaction, on utilise un solvant choisi parmi le groupe comprenant les hydrocarbures aliphatiques, les hydrocarbures cycloaliphatiques, les hydrocarbures aromatiques, les éthers, les esters ou les hydrocarbures chlorés.

3. Procédé suivant les revendications 1 et 2, caractérisé en ce qu'on effectue la distillation du mélange réactionnel, qui est éventuellement effectuée après une extraction avec l'eau, sous des pressions se situant dans l'intervalle allant de 0,1 à 500 mbars.

4. Procédé suivant les revendications 1 à 3, caractérisé en ce qu'on extrait le mélange réactionnel avec 1 à 3 fois la quantité pondérale d'eau en obtenant une phase aqueuse contenant un acide carboxylique, ainsi qu'un mélange réactionnel essentiellement exempt d'acide carboxylique, ce dernier mélange étant séparé en ses composants par distillation.

5. Procédé suivant la revendication 4, caractérisé en ce qu'on extrait la phase aqueuse provenant de l'extraction dans une deuxième extraction avec 1 à 3 fois la quantité pondérale (calculé sur la phase aqueuse à extraire) d'un solvant donnant une non-miscibilité avec l'eau.

6. Procédé suivant les revendications 4 et 5, caractérisé en ce qu'on effectue en continu la distillation de la phase organique provenant de l'extraction du mélange réactionnel avec l'eau et, en l'occurrence, dans une première unité de distillation, on sépare le solvant comme produit de tête puis, dans une deuxième unité de distillation, on sépare l'alcène n'ayant par réagi comme produit de tête et, dans une troisième unité de distillation, on sépare l'alkyl-oxiranne formé comme produit de tête.